# EUROPEAN PATENT APPLICATION

(11) **EP 4 603 013 A1**
(43) Date of publication of application: **20.08.2025**
(21) Application number: 23894453.2
(22) Date of filing: 10.11.2023
(51) Int. Cl.: A61B 5/11, A47C 27/14, A47G 9/02, A61B 5/08, A61B 5/022, A61B 5/113, A61B 5/0245, G01L 5/00

(54) **SENSOR SHEET AND CUSHION**

(30) Priority: 24.11.2022 JP 2022187497
(71) Applicant: NISHIKAWA CO., LTD., Tokyo 103-0006 (JP)
(72) Inventor: NONOMURA, Takuto, Tokyo 103-0006 (JP); IKEDA, Sho, Tokyo 103-0006 (JP); SHIMADA, Saki, Tokyo 103-0006 (JP); AOKI, Mari, Tokyo 103-0006 (JP)
(74) Representative: Viering, Jentschura & Partner mbB Patent- und Rechtsanwälte
(86) International application number: PCT/JP2023/040635
(87) International publication number: WO 2024/111444

(57) **Abstract**

A sensor sheet according to one embodiment is a sensor sheet that is attached to a cushion body including a core material made of a flexible material and a cover fabric that accommodates the core material and that is attachable to and detachable from the core material, and that acquires vital data of a user of the cushion body. The sensor sheet includes a sheet-shaped fabric; a thread-shaped sensor embroidered on the sheet-shaped fabric; and a fixing portion that fixes the sheet-shaped fabric to the cover fabric. The fixing portion is attachable to and detachable from the cover fabric.

## Description

### Technical Field

The present disclosure relates to a sensor sheet and a cushion body.

Priority is claimed on Japanese Patent Application No. 2022-187497, filed on November 24, 2022, the entire content of which is incorporated herein by reference.

### Background Art

Japanese Unexamined Patent Publication No. 2021-185996 discloses bedding on which the body of a user is placed. The bedding includes a cover fabric and a plurality of thread-shaped sensors embroidered on the cover fabric. The thread-shaped sensors acquire time-series data of a voltage value. The time-series data is used to acquire data regarding the breathing, heartbeat, sleep time, or the like of the user.

### Citation List

### Patent Literature

Patent Literature 1: Japanese Unexamined Patent Publication No. 2021-185996

### Summary of Invention

### Technical Problem

In addition to being capable of suppressing misalignment of the thread-shaped sensors, the bedding described above is required to allow the cover fabric to be easily washed. The thread-shaped sensors may be vulnerable to water. Therefore, when the cover fabric is washed, it is desirable to remove the thread-shaped sensors from the cover fabric. However, in the bedding described above, since the thread-shaped sensors are directly embroidered on the cover fabric, the thread-shaped sensors cannot be easily removed from the cover fabric. The bedding described above includes the plurality of thread-shaped sensors to acquire a wide range of data (vital data of the user). However, when the number of the thread-shaped sensors is large, there is a possibility that the number of connectors and the like increases and an electrical system such as the connectors become complicated. Therefore, it is desirable to acquire a wide range of vital data using a small number of the thread-shaped sensors.

An object of the present disclosure is to provide a sensor sheet and a cushion body that can suppress misalignment, allows a cover fabric to be easily washed, and can acquire a wide range of vital data using a small number of thread-shaped sensors.

### Solution to Problem

(1) A sensor sheet according to the present disclosure is attached to a cushion body including a core material made of a flexible material and a cover fabric that accommodates the core material and that is attachable to and detachable from the core material. The sensor sheet acquires vital data of a user of the cushion body. The sensor sheet includes a sheet-shaped fabric; a thread-shaped sensor embroidered on the sheet-shaped fabric; and a fixing portion that fixes the sheet-shaped fabric to at least one of the core material and the cover fabric. The fixing portion is attachable to and detachable from the at least one of the core material and the cover fabric.
   The sensor sheet according to the present disclosure includes the fixing portion that fixes the sheet-shaped fabric to the at least one of the core material and the cover fabric. By fixing the sheet-shaped fabric to the core material or the cover fabric using the fixing portion, misalignment of the sensor sheet when the body of the user of the cushion body is placed on the cushion body can be suppressed. For example, when the thread-shaped sensor is directly embroidered on the cover fabric, the thread-shaped sensor cannot be easily removed from the cover fabric, and therefore, the cover fabric cannot be washed. On the other hand, in the sensor sheet, the fixing portion is attachable to and detachable from the at least one of the core material and the cover fabric, and therefore, the sensor sheet can be easily removed from the cover fabric or the core material removed from the cover fabric. Therefore, the cover fabric can be easily washed. The sensor sheet includes the sheet-shaped fabric and the thread-shaped sensor embroidered on the sheet-shaped fabric. For example, one thread-shaped sensor can be attached by being embroidered such that the one thread-shaped sensor spreads out two-dimensionally on the sheet-shaped fabric. As a result, a wide range of vital data can be acquired by the thread-shaped sensor, and the number of the thread-shaped sensors can be reduced. Further, an electrical system such as a connector can be simplified.
(2) In (1) above, the core material may have an upper surface on which a body of the user is placed, a lower surface facing opposite the upper surface, and side surfaces connecting the upper surface and the lower surface. The fixing portion may fix the sheet-shaped fabric to the cover fabric at positions between the side surfaces of the core material and the cover fabric. For example, when the fixing portion fixes the sheet-shaped fabric to the cover fabric at positions between the upper surface on which the body of the user is placed and the cover fabric, there is a possibility that the fixing portion comes into contact with the body of the user when the body of the user is placed on the cushion body, thereby causing discomfort to the user. On the other hand, when the fixing portion fixes the sheet-shaped fabric to the cover fabric between the side surfaces of the core material and the cover fabric, causing discomfort to the user can be avoided.
(3) In (1) or (2) above, the core material and the cover fabric may have a rectangular shape in a plan view. The cover fabric may include a plurality of fixed portions to which the fixing portion is fixed. The plurality of fixed portions may be aligned along a longitudinal direction of the cover fabric. When the body of the user is placed on the cushion body, for example, the body of the user is placed along the longitudinal direction of the cover fabric. When the plurality of fixed portions are aligned along the longitudinal direction of the cover fabric, the fixing position of the sensor sheet can be changed along the longitudinal direction of the cover fabric. Accordingly, for example, the position of the sensor sheet in the longitudinal direction of the cover fabric can be adjusted according to the physique of the user. Since the sensor sheet can be disposed at an appropriate position according to the user, the vital data of the user can be more accurately acquired.
(4) In any one of (1) to (3) above, the fixing portion may include snap buttons. The fixing portion may fix the sheet-shaped fabric to the at least one of the core material and the cover fabric via the snap buttons. In this case, the sensor sheet is more easily attachable to and detachable from the cover fabric via the snap buttons. Compared to when the fixing portion includes hook-and-loop fasteners instead of the snap buttons, discomfort when the fixing portion comes into contact with the body of the user can be reduced.
(5) In any one of (1) to (4) above, the sheet-shaped fabric may include an inner fabric on which the thread-shaped sensor is embroidered, and an outer fabric that accommodates the inner fabric. The outer fabric may be waterproof. The thread-shaped sensor may be vulnerable to moisture, and in this case, when the thread-shaped sensor comes into contact with water, there is a possibility that the detection accuracy of the thread-shaped sensor decreases. When the inner fabric on which the thread-shaped sensor is embroidered is accommodated in the outer fabric that is waterproof, the contact of the thread-shaped sensor with moisture can be suppressed. Therefore, a decrease in the detection accuracy of the thread-shaped sensor can be suppressed.
(6) In any one of (1) to (5) above, the thread-shaped sensor may include a curved portion having a curved shape in a plan view. In the curved portion at which the thread-shaped sensor has a curved shape, the detection accuracy is higher than, for example, in a portion at which the thread-shaped sensor has a linear shape. When the thread-shaped sensor includes the curved portion having a curved shape in a plan view, the detection accuracy of the thread-shaped sensor can be improved.
(7) In any one of (1) to (6) above, the sensor sheet may further include another fixing portion different from the fixing portion. The sheet-shaped fabric may have, in a plan view, a major axis extending in a longitudinal direction, and a minor axis extending in a lateral direction and intersecting the major axis. The fixing portion may be connected to one end of the sheet-shaped fabric in the longitudinal direction. The other fixing portion may be connected to the other end of the sheet-shaped fabric in the longitudinal direction. The core material may have an upper surface on which a body of the user is placed, a lower surface facing opposite the upper surface, and side surfaces connecting the upper surface and the lower surface. In a state where the sheet-shaped fabric is disposed on the upper surface of the core material, the sheet-shaped fabric may be fixed to the core material by fixing the fixing portion and the other fixing portion to each other on the lower surface of the core material. In this case, for example, by fixing the sheet-shaped fabric to the core material using the fixing portion and the other fixing portion that are belt-shaped members, misalignment of the sensor sheet when the body of the user of the cushion body is placed on the cushion body can be suppressed. Since the fixing portion and the other fixing portion are attachable to and detachable from the core material, the sensor sheet can be easily removed from the core material removed from the cover fabric.
(8) In (7) above, the fixing portion may include a first fixing member provided on one end side of the fixing portion in the longitudinal direction. The other fixing portion may include a second fixing member provided on the other end side of the other fixing portion in the longitudinal direction. The fixing portion and the other fixing portion may be fixed to each other via the first fixing member and the second fixing member. In this case, the fixing portion and the other fixing portion are more firmly fixed to the core material by the first fixing member and the second fixing member.
(9) In (8) above, each of the fixing portion and the other fixing portion may include a support at a portion adjacent to the sheet-shaped fabric. The support of the fixing portion may be provided at an end portion of the fixing portion on a side opposite the first fixing member. The support of the other fixing portion may be provided at an end portion of the other fixing portion on a side opposite the second fixing member. In a state where the sensor sheet is attached to the core material, the support of the fixing portion and the support of the other fixing portion may face the side surfaces of the core material. In this case, when the body of the user is placed on the sensor sheet, the deformation of the fixing portion and the other fixing portion on the side surfaces of the core material can be suppressed. The occurrence of gaps between the side surface of the core material and the fixing portion and between the side surface of the core material and the other fixing portion can be suppressed, and misalignment of the sensor sheet can be more reliably suppressed.
(10) A cushion body according to the present disclosure includes the sensor sheet according to any one of (1) to (9) above; the core material; and the cover fabric that accommodates the core material.

In the cushion body according to the present disclosure, the sensor sheet includes the fixing portion for fixing the sheet-shaped fabric on which the thread-shaped sensor is embroidered. Similarly to the sensor sheet described above, misalignment of the sensor sheet when the body of the user of the cushion body is placed on the cushion body can be suppressed. In the cushion body, the fixing portion is attachable to and detachable from at least one of the core material and the cover fabric, and therefore, the sensor sheet can be easily removed from the cover fabric or the core material removed from the cover fabric. Therefore, the cover fabric can be easily washed. Further, in the cushion body, similarly to the sensor sheet described above, for example, one thread-shaped sensor can be attached by being embroidered such that the one thread-shaped sensor spreads out two-dimensionally on the sheet-shaped fabric. An electrical system such as a connector can be simplified, and a wide range of vital data can be acquired by a small number of the thread-shaped sensors.

### Advantageous Effects of Invention

According to the present disclosure, it is possible to suppress misalignment, allow the cover fabric to be easily washed, and acquire a wide range of vital data using a small number of the thread-shaped sensors.

### Brief Description of Drawings

[FIG. 1A] FIG. 1A is a schematic perspective view showing a cushion body according to one embodiment.
[FIG. 1B] FIG. 1B is a schematic perspective view showing a core material constituting the cushion body shown in FIG. 1A.
[FIG. 2] FIG. 2 is a schematic plan view showing a sensor sheet according to one embodiment.
[FIG. 3] FIG. 3 is an enlarged schematic view showing a fixing portion of the sensor sheet and a fixed portion of a cover fabric.
[FIG. 4] FIG. 4 is a partial enlarged schematic view showing the sensor sheet shown in FIG. 2.
[FIG. 5] FIG. 5 is a block diagram showing a sensing system according to one embodiment.
[FIG. 6] FIG. 6 is a schematic plan view showing a sensor sheet according to a modification example.
[FIG. 7] FIG. 7 is a schematic perspective view showing a core material to which the sensor sheet shown in FIG. 6 is attached.
[FIG. 8A] FIG. 8A is a schematic plan view showing a sensor sheet according to a modification example.
[FIG. 8B] FIG. 8A is a schematic plan view showing a sensor sheet according to another modification example.

### Description of Embodiments

Hereinafter, an embodiment of a sensor sheet and a cushion body according to the present disclosure will be described with reference to the drawings. In the description of the drawings, the same or corresponding elements are denoted by the same reference signs, and duplicate descriptions will be omitted as appropriate. For ease of understanding, the drawings may be depicted in a partially simplified or exaggerated manner, and dimensional ratios and the like are not limited to those shown in the drawings.

The cushion body to which the sensor sheet according to the present disclosure will be described. The cushion body is, for example, bedding. The "bedding" refers to an article with which the body of a user comes into contact when the user rests or sleeps. The "bedding" typically refers to laying bedding such as a mattress or a futon, a cushion, a bed pad, a floor cushion, or a pillow. The cushion body may be, for example, a seat that is mounted in a vehicle.

The cushion body is used while being placed on a placement surface. The "placement surface" is a surface on which the cushion body is placed, and is, as one example, a floor. The cushion body has an upper surface on which the body of the user is placed; a lower surface facing opposite the upper surface; and a side surface connecting the upper surface and the lower surface. The "cushion body" is placed on the placement surface such that the lower surface of the cushion body comes into contact with the placement surface. The body of the user is placed on the upper surface of the cushion body.

The cushion body includes a core material made of a flexible material, and a cover fabric that accommodates the core material. The cover fabric is attachable to and detachable from the core material. The "flexible material" refers to a cushioning material that deforms when subjected to a load due to the body of the user being placed on the flexible material. The flexible material is, for example, cloth. The "cover fabric" refers to a bag-shaped fabric constituting the cushion body.

The "user" refers to a person who uses the cushion body by bringing his or her body into contact with the cushion body. For example, when the cushion body is a mattress, the user is a person who places his or her body on the mattress. The user is, for example, a person who lies on a purchased cushion body, or a person who lies on a cushion body to purchase the cushion body.

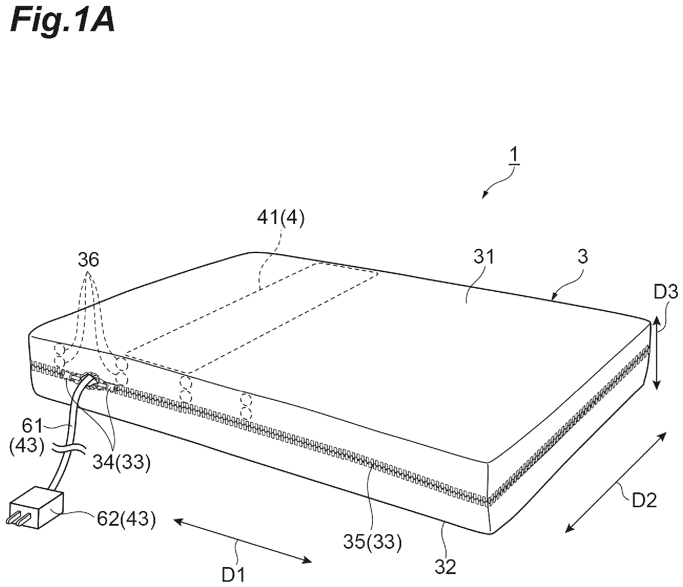

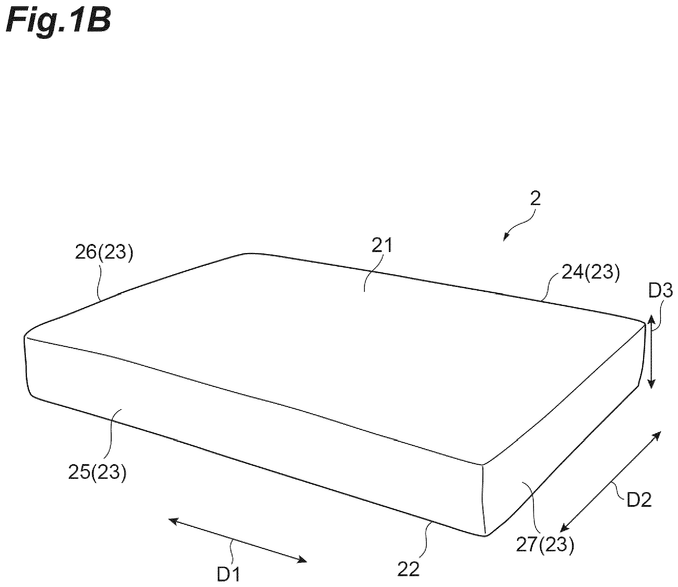

FIG. 1A is a schematic perspective view showing a cushion body 1 according to one embodiment. FIG. 1B is a schematic perspective view showing a core material 2 constituting the cushion body shown in FIG. 1A. As shown in FIG. 1A, the cushion body 1 has a rectangular shape in a plan view. The cushion body 1 extends in a longitudinal direction D1 and a lateral direction D2 orthogonal to the longitudinal direction D1. The cushion body 1 has a thickness in a thickness direction D3 orthogonal to both the longitudinal direction D1 and the lateral direction D2.

A length of the cushion body 1 in the longitudinal direction D1 is, for example, 120 cm or more and 210 cm or less (as one example, 195 cm). A length of the cushion body 1 in the lateral direction D2 is, for example, 70 cm or more and 180 cm or less (as one example, 97 cm). A length of the cushion body 1 in the thickness direction D3 is, for example, 3 cm or more and 40 cm or less (as one example, 9 cm).

In the present embodiment, the cushion body 1 is a mattress. The user of the cushion body 1 places his or her body on the cushion body 1. At this time, a direction in which the body of the lying user extends (namely, a direction in which the head and the legs of the user are connected to each other) coincides with, for example, the longitudinal direction D1 of the cushion body 1.

As shown in FIG. 1B, the cushion body 1 includes the core material 2 accommodated inside a cover fabric 3 to be described later. In the present embodiment, the core material 2 has a rectangular shape in a plan view. The core material 2 includes, for example, a content and a bag that accommodates the content. The content is, for example, urethane foam or polyester. The material of the bag is, for example, cotton or polyester.

The core material 2 has a rectangular parallelepiped shape. The core material 2 has an upper surface 21 on which the body of the user is placed, and a lower surface 22 facing opposite the upper surface 21. The upper surface 21 is a surface facing one side (upper side in FIG. 1B) in the thickness direction D3. The lower surface 22 is a surface facing the other side (lower side in FIG. 1B) in the thickness direction D3.

The core material 2 has a plurality of side surfaces 23 connecting the upper surface 21 and the lower surface 22. Hereinafter, for convenience of description, a surface facing one side (upper right side in FIG. 1B) in the lateral direction D2 among the plurality of side surfaces 23 is referred to as a first side surface 24, and a surface facing the other side (lower left side in FIG. 1B) in the lateral direction D2 among the plurality of side surfaces 23 is referred to as a second side surface 25. The first side surface 24 and the second side surface 25 extend in the longitudinal direction D1 and the thickness direction D3. The first side surface 24 and the second side surface 25 are orthogonal to the lateral direction D2.

A surface facing one side (upper left side in FIG. 1B) in the longitudinal direction D1 among the plurality of side surfaces 23 is referred to as a third side surface 26, and a surface facing the other side (lower right side in FIG. 1B) in the longitudinal direction D1 among the plurality of side surfaces 23 is referred to as a fourth side surface 27. The third side surface 26 and the fourth side surface 27 extend in the lateral direction D2 and the thickness direction D3. The third side surface 26 and the fourth side surface 27 are orthogonal to the longitudinal direction D1.

As shown in FIG. 1A, the cushion body 1 includes the cover fabric 3. In the present embodiment, the cover fabric 3 has a bag shape. The cover fabric 3 has a rectangular shape in a plan view. The cover fabric 3 includes, for example, a front fabric 31 covering the upper surface 21 of the core material 2; a back fabric 32 covering the lower surface 22 of the core material 2; and an opening and closing member 33 connecting the front fabric 31 and the back fabric 32 to each other. The opening and closing member 33 is provided at a position on the cover fabric 3 which faces the side surfaces 23 of the core material 2. When viewed in the thickness direction D3, the opening and closing member 33 is located outside the side surfaces 23 of the core material 2. The cover fabric 3 is attachable to and detachable from the core material 2. "Being attachable to and detachable from the core material" includes a case in which the cover fabric is turned over with respect to the core material. "Being attachable to and detachable from the core material" includes a case in which the cover fabric is turned over to expose at least a part of the core material.

The opening and closing member 33 is, as one example, a so-called double slider. The opening and closing member 33 includes two slide members 34 and an opening and closing portion 35 that opens and closes the front fabric 31 and the back fabric 32 when the slide members 34 are slid. The front fabric 31 is separated from the back fabric 32 by opening the front fabric 31 and the back fabric 32, and the front fabric 31 is joined to the back fabric 32 by closing the front fabric 31 and the back fabric 32.

For example, the front fabric 31 and the back fabric 32 can be opened by sliding one slide member 34 along the opening and closing portion 35 in one direction (clockwise direction in FIG. 1A) and sliding the other slide member 34 along the opening and closing portion 35 in the other direction (counterclockwise direction in FIG. 1A).

The front fabric 31 and the back fabric 32 can be closed by sliding the one slide member 34 along the opening and closing portion 35 in the other direction and sliding the other slide member 34 along the opening and closing portion 35 in the one direction. The opening and closing member 33 is also referred to as a single slider. In this case, the opening and closing member 33 includes one slide member 34 and the opening and closing portion 35.

The cover fabric 3 includes fixed portions 36 to which fixing portions 71 and 72 to be described later are fixed. The fixed portions 36 are provided a back surface of the cover fabric 3. In the present embodiment, the cover fabric 3 includes a plurality of the fixed portions 36. The fixed portions 36 are provided, for example, at positions on the front fabric 31 which face the first side surface 24 of the core material 2. For example, the fixed portions 36 are provided at positions on the front fabric 31 which face the second side surface 25 of the core material 2.

The plurality (as one example, eight) of fixed portions 36 are aligned along the longitudinal direction D1 of the cover fabric 3. In the example of FIG. 1A, two fixed portions 36 are aligned along the thickness direction D3. Four rows, each being composed of two fixed portions 36, are aligned along the longitudinal direction D1. For example, the fixed portions 36 are female parts of snap buttons 73 to be described later.

A sensor sheet 4 is attached to the cushion body 1. The sensor sheet 4 acquires vital data of the user of the cushion body 1. The vital data includes, for example, information regarding the heartbeat, breathing, and body movement of the user. The contents of the vital data can be changed as appropriate. The vital data may include, for example, information regarding blood pressure.

The sensor sheet 4 is disposed inside the cover fabric 3 having a bag shape. More specifically, the sensor sheet 4 is disposed between the core material 2 and the cover fabric 3. When the sensor sheet 4 is attached to the cushion body 1, the sensor sheet 4 is disposed to extend in the lateral direction D2 of the cushion body 1.

The sensor sheet 4 is disposed, for example, at a position where the position of the sensor sheet 4 in the longitudinal direction D1 faces the heart of the user. As one example, the sensor sheet 4 is attached at a position (position on the lower right side in FIG. 1A) spaced apart by a certain distance along the longitudinal direction D1 of the cushion body 1 from the position where the head of the user is placed. The attachment position of the sensor sheet 4 with respect to the cushion body 1 can be changed. The distance from the position where the head of the user is placed to the position where the sensor sheet 4 is attached may be, for example, 40 cm or more and 50 cm or less (as one example, 50 cm).

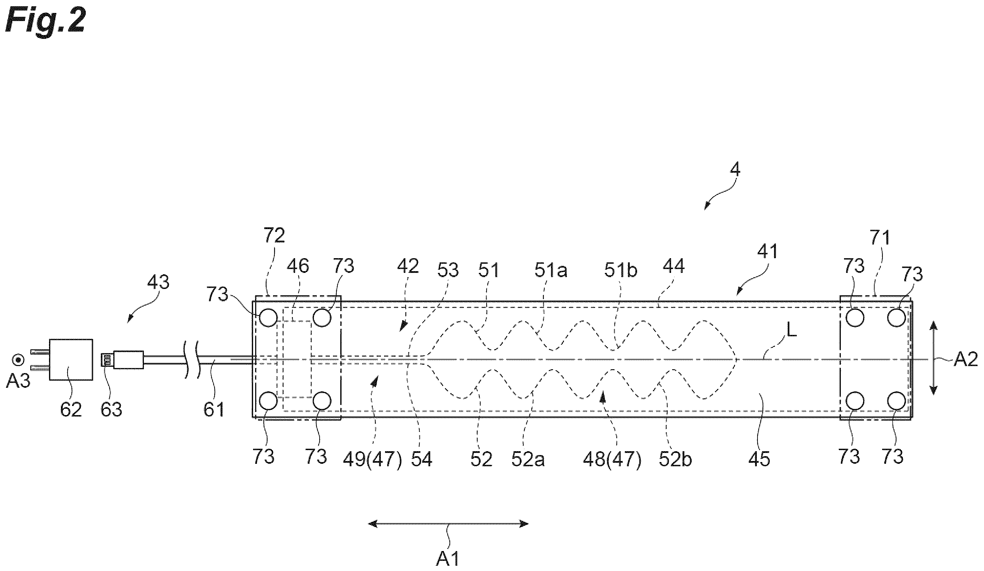

The sensor sheet 4 will be described in more detail. FIG. 2 is a schematic plan view showing the sensor sheet 4 according to one embodiment. The sensor sheet 4 has a rectangular shape extending in one direction. The sensor sheet 4 includes a sheet-shaped fabric 41, a sensor unit 42, and a power supply unit 43.

The sheet-shaped fabric 41 is a sheet-shaped fabric having a rectangular shape in a plan view. The sheet shape refers to a shape extending in each of one direction and another direction intersecting the one direction. Namely, the sheet shape represents a state where the shape is spread out two-dimensionally in two directions. Hereinafter, a longitudinal direction of the sheet-shaped fabric 41 is referred to as a first direction A1, and a lateral direction of the sheet-shaped fabric 41 is referred to as a second direction A2. A direction orthogonal to both the first direction A1 and the second direction A2 is referred to as a third direction A3.

For example, a length of the sheet-shaped fabric 41 in the first direction A1 is longer than the length of the cushion body 1 in the lateral direction D2. In a state where the sensor sheet 4 is attached to the cushion body 1, a central portion of the sheet-shaped fabric 41 is located on the upper surface 21 of the core material 2. In a state where the sensor sheet 4 is attached to the cushion body 1, both end portions of the sheet-shaped fabric 41 in the first direction A1 face the side surfaces 23 (the first side surface 24 and the second side surface 25) of the core material 2. The central portion of the sheet-shaped fabric 41 faces the upper surface 21 of the core material 2. Both the end portions of the sheet-shaped fabric 41 face the side surfaces 23 of the core material 2 in a state where both the end portions are folded downward with respect to the central portion.

The length of the sheet-shaped fabric 41 in the first direction A1 is, for example, 76 cm or more and 260 cm or less (as one example, 113 cm). A length of the sheet-shaped fabric 41 in the second direction A2 is, for example, 10 cm or more and 70 cm or less (as one example, 23 cm).

The sheet-shaped fabric 41 has, for example, stretchability. The sheet-shaped fabric 41 may have, for example, such high stretchability as not to impair the sleeping comfort of the user. From the viewpoint of not impairing the sleeping comfort of the user, the sheet-shaped fabric 41 may have stretchability less than or equal to a certain level. A thread-shaped sensor 47 is embroidered on the sheet-shaped fabric 41. The thread-shaped sensor 47 acquires vital data. The sheet-shaped fabric 41 may have, for example, such low stretchability as not to interfere with the acquisition of the vital data by the thread-shaped sensor 47. From the viewpoint of accurately acquiring the vital data, the sheet-shaped fabric 41 may have stretchability less than or equal to a certain level.

The stretchability of the sheet-shaped fabric 41 may be evaluated, as one example, by the elongation elastic modulus. The elongation elastic modulus of the sheet-shaped fabric 41 is, for example, 15% or more and 35% or less (as one example, 27.4%). For example, the method of JIS L1096 can be used as a method for measuring an elongation elastic modulus.

For example, the sheet-shaped fabric 41 has a triple-layer structure in which three pieces of fabric are layered along the third direction A3. The sheet-shaped fabric 41 includes an inner fabric 44 and an outer fabric 45 having a bag shape that accommodates the inner fabric 44. The inner fabric 44 and the outer fabric 45 are sewn to each other inside the outer fabric 45. As one example, an inner peripheral portion of the inner fabric 44 is sewn to the outer fabric 45. A length of the inner fabric 44 in the first direction A1 is shorter than a length of the outer fabric 45 in the first direction A1.

For example, the outer fabric 45 is made of a waterproof material. As one example, the outer fabric 45 may be a fabric, the back surface of which (an inner surface of the outer fabric 45) is laminated or resin-treated. The outer fabric 45 may be a membrane fabric. The outer fabric 45 may be water-repellent.

The sensor unit 42 includes a main unit 46 that acquires vital data, and the thread-shaped sensor 47 that detects the vital data. The main unit 46 is fixed to an end portion (left end in FIG. 2) of the inner fabric 44 in the first direction A1. The main unit 46 outputs the acquired vital data to a device external to the sensor sheet 4. The main unit 46 has, for example, an electrical function achieved by an electronic component (or an electrical component). A functional configuration of the main unit 46 will be described later.

The thread-shaped sensor 47 detects the vital data of the user of the cushion body 1. The thread-shaped sensor 47 is, as one example, a piezoelectric sensor. However, the type of the thread-shaped sensor 47 is not particularly limited. For example, the thread-shaped sensor 47 is a single sensor, and one thread-shaped sensor 47 is fixed by being embroidered such that the one thread-shaped sensor 47 spreads out two-dimensionally on the sheet-shaped fabric 41. In the example of FIG. 2, the thread-shaped sensor 47 has a shape symmetrical with respect to a center line L. The center line L passes through the center of the sheet-shaped fabric 41 in the second direction A2. The center line L extends in the first direction A1.

The thread-shaped sensor 47 includes a curved portion 48 having a curved shape in a plan view, and a linear portion 49 having a linear shape in a plan view. For example, the curved portion 48 has a wave shape in a plan view. The "wave shape" includes a sine wave shape and a circular wave shape. The curved portion 48 includes a first wave-shaped portion 51 and a second wave-shaped portion 52 aligned along the second direction A2.

The first wave-shaped portion 51 is located on one side (upper side in FIG. 2) of the center line L in the second direction A2. The first wave-shaped portion 51 includes peak portions 51a and valley portions 51b that are alternately aligned along the first direction A1. The first wave-shaped portion 51 includes a plurality of the peak portions 51a and a plurality of the valley portions 51b. The peak portions 51a projects toward the one side in the second direction A2 with respect to the center line L. The valley portions 51b are portions recessed toward the center line L. In the example of FIG. 2, the first wave-shaped portion 51 includes five peak portions 51a and four valley portions 51b.

The second wave-shaped portion 52 is located on the other side of the center line L in the second direction A2 (the side opposite the first wave-shaped portion 51 when viewed from the center line L). The second wave-shaped portion 52 includes peak portions 52a and valley portions 52b that are alternately aligned along the first direction A1. The second wave-shaped portion 52 includes a plurality of the peak portions 52a and a plurality of the valley portions 52b. The peak portions 52a projects toward the other side in the second direction A2 with respect to the center line L. The valley portions 52b are portions recessed toward the center line L. In the example of FIG. 2, the second wave-shaped portion 52 includes five peak portions 52a and four valley portions 52b. The first wave-shaped portion 51 and the second wave-shaped portion 52 are connected to each other at the end portion (right end in FIG. 2) of the sheet-shaped fabric 41A on the side opposite the main unit 46.

For example, a length of the first wave-shaped portion 51 in the first direction A1 and a length of the second wave-shaped portion 52 in the first direction A1 are equal to each other. The length of the first wave-shaped portion 51 and the second wave-shaped portion 52 in the first direction A1 is, for example, 30 cm or more and 180 cm or less (as one example, 56 cm).

A length of the first wave-shaped portion 51 in the second direction A2 and a length of the second wave-shaped portion 52 in the second direction A2 are equal to each other. The length of the first wave-shaped portion 51 and the second wave-shaped portion 52 in the second direction A2 is, for example, 2 cm or more and 30 cm or less (as one example, 6 cm). Incidentally, the length of the first wave-shaped portion 51 in the second direction A2 is a length along the second direction A2 from tops of the peak portions 51a to bottoms of the valley portions 51b. The length of the second wave-shaped portion 52 in the second direction A2 is a length along the second direction A2 from tops of the peak portions 52a to bottoms of the valley portions 52b.

The linear portion 49 is a portion extending along the first direction A1. The linear portion 49 includes a first linear portion 53 and a second linear portion 54 aligned along the second direction A2. The first linear portion 53 is located on the one side of the center line L in the second direction A2. One end of the first linear portion 53 in the first direction A1 is connected to an end portion (left end in FIG. 2) of the first wave-shaped portion 51 in the first direction A1. An end portion of the first linear portion 53 on the side opposite the first wave-shaped portion 51 is connected to the main unit 46.

The second linear portion 54 is located on the other side of the center line L in the second direction A2 (the side opposite the first linear portion 53 when viewed from the center line L). One end of the second linear portion 54 in the first direction A1 is connected to an end portion of the second wave-shaped portion 52 in the first direction A1. An end portion of the second linear portion 54 on the side opposite the second wave-shaped portion 52 is connected to the main unit 46.

For example, a length of the first linear portion 53 in the first direction A1 and a length of the second linear portion 54 in the first direction A1 are equal to each other. The length of the first linear portion 53 in the first direction A1 is, for example, 1 cm or more and 50 cm or less (as one example, 24 cm). The length of the second linear portion 54 in the first direction A1 is, for example, 1 cm or more and 50 cm or less (as one example, 24 cm).

In the thread-shaped sensor 47 that is a single sensor, the first wave-shaped portion 51, the second wave-shaped portion 52, the first linear portion 53, and the second linear portion 54 are connected to each other. The thread-shaped sensor 47 is attached to spread out in both the first direction A1 and the second direction A2 at the position where the curved portion 48 is disposed.

The power supply unit 43 supplies electric power to the main unit 46. The sensor unit 42 is activated when the power supply unit 43 supplies electric power to the main unit 46. The power supply unit 43 includes, for example, a power cord 61 and a power plug 62. One end of the power cord 61 is connected to the main unit 46 of the sensor unit 42. A connector 63 connected to the power plug 62 is provided at the other end of the power cord 61. The power cord 61 and the power plug 62 are connected to each other via the connector 63. The type of the connector 63 is not particularly limited. The connector 63 may be compliant with, for example, the Universal Serial Bus (USB) Type-C (registered trademark) standard.

The power plug 62 is inserted into, for example, a household outlet. The power plug 62 may be connected to, for example, a battery. Unlike the example of FIG. 2, the power supply unit 43 may not include the power plug 62. In this case, for example, the connector 63 of the power cord 61 is inserted into the battery. The sensor unit 42 is activated when the power supply unit 43 supplies electric power from the battery to the main unit 46.

The sensor sheet 4 includes the fixing portions 71 and 72. For example, the fixing portions 71 and 72 are portions for fixing the sheet-shaped fabric 41 to the cover fabric 3. The fixing portions 71 and 72 are attachable to and detachable from the cover fabric 3. The fixing portion 71 is provided on one end side of the sheet-shaped fabric 41 in the first direction A1. The fixing portion 72 is provided on the other end side of the sheet-shaped fabric 41 in the first direction A1.

Each of the fixing portions 71 and 72 includes the snap button 73. The snap button 73 of each of the fixing portions 71 and 72 is, for example, a male part of the snap button 73 that locks to the female part of the snap button 73 constituting the fixed portion 36 of the cover fabric 3. Each of the fixing portions 71 and 72 includes a plurality (as one example, four) of the snap buttons 73.

In the fixing portion 71, two snap buttons 73 aligned along the first direction A1 are provided on the one end side of the sheet-shaped fabric 41 in the second direction A2. In the fixing portion 71, two snap buttons 73 aligned along the first direction A1 are provided on the other end side of the sheet-shaped fabric 41 in the second direction A2. In the fixing portion 71, the plurality of snap buttons 73 are disposed to form a polygonal shape. However, the plurality of snap buttons 73 may be disposed to be aligned on a straight line. In the present embodiment, the four snap buttons are disposed to form a quadrilateral shape (as one example, rectangular shape). The disposition of the snap buttons 73 in the fixing portion 72 are similar to the disposition of the snap buttons 73 in the fixing portion 71. Therefore, the description regarding the disposition of the snap buttons 73 in the fixing portion 72 will be omitted.

In a state where the sensor sheet 4 is attached to the cushion body 1, for example, the first direction A1 of the sensor sheet 4 coincides with the lateral direction D2 of the cushion body 1. At this time, the second direction A2 of the sensor sheet 4 coincides with the longitudinal direction D1 of the cushion body 1. A portion of the sheet-shaped fabric 41 in which the fixing portions 71 and 72 are not provided is disposed between the upper surface 21 of the core material 2 and the cover fabric 3. The fixing portion 71 is disposed between the first side surface 24 of the core material 2 and the cover fabric 3. The fixing portion 72 is disposed between the second side surface 25 of the core material 2 and the cover fabric 3.

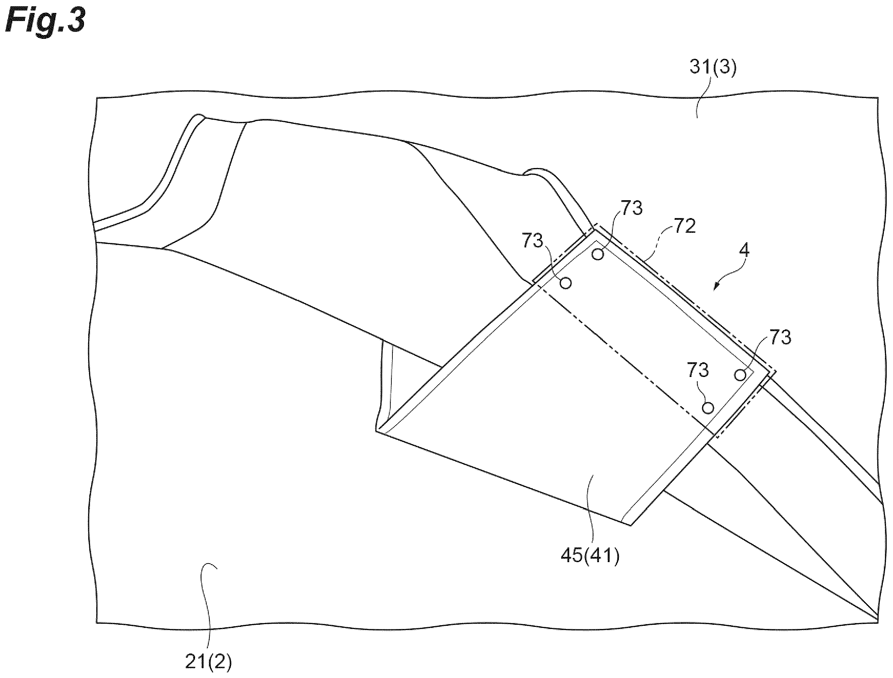

FIG. 3 is an enlarged schematic view showing the fixing portion of the sensor sheet 4 and the cover fabric 3. FIG. 3 illustrates a state where the front fabric 31 of the cover fabric 3 is turned over to an upper surface 21 side of the core material 2 in a state where the sensor sheet 4 is fixed to the cover fabric 3. The fixing portions 71 and 72 fix the sheet-shaped fabric 41 to the cover fabric 3 at positions between the side surfaces 23 of the core material 2 and the cover fabric 3. For example, the fixing portions 71 and 72 fix the sheet-shaped fabric 41 to the cover fabric 3 via the snap buttons 73.

In a state where the core material 2 is accommodated in the cover fabric 3, each of two snap buttons 73 is fixed to the fixed portion 36 (see FIG. 1A) of the cover fabric 3. As described above, the fixed portion 36 includes two snap buttons 73 aligned along the thickness direction D3 on the cover fabric 3. The two snap buttons 73 in the fixing portion 72 is locked to the respective two snap buttons 73 in the cover fabric 3. Similarly to the fixing portion 72, the snap buttons 73 in the fixing portion 71 are also locked to the snap buttons 73 of the fixed portions 36 in the cover fabric 3.

By fixing the sensor sheet 4 to the cover fabric 3 using the plurality of snap buttons 73, misalignment of the sensor sheet 4 when the body of the user is placed on the cushion body 1 can be more reliably suppressed. Since the fixing portions 71 and 72 fix the sensor sheet 4 to the cover fabric 3 via the snap buttons 73, the sensor sheet 4 can be easily removed from the cover fabric 3.

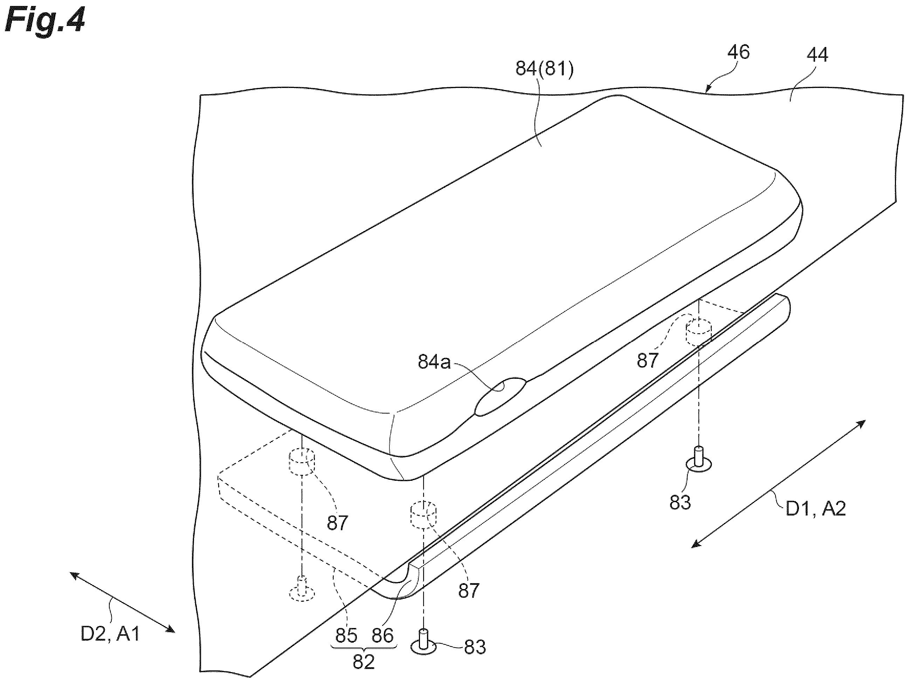

FIG. 4 is an enlarged perspective view showing the main unit 46. The main unit 46 includes an electrical function portion 81, an attachment member 82, and screws 83. The electrical function portion 81 is a portion having an electrical function achieved by an electronic component (or an electrical component). For example, the electrical function portion 81 includes an electronic component and a housing 84 that accommodates the electronic component. The electronic component is electrically connected to the thread-shaped sensor 47.

The housing 84 includes a socket 84a into which the one end of the power cord 61 is inserted. The socket 84a is formed, for example, on a side surface (as one example, a surface facing the first direction A1) of the housing 84. The housing 84 has insertion holes into which the screws 83 are inserted. The housing 84 has a plurality (as one example, four) of the insertion holes. A screw groove into which the screw 83 is screwed is provided on an inner surface of each insertion hole.

The attachment member 82 is a member for attaching the electrical function portion 81 to the sheet-shaped fabric 41. The attachment member 82 includes a plate-shaped portion 85 and a protruding portion 86. The plate-shaped portion 85 is a plate-shaped portion extending in the first direction A1 and the second direction A2. The plate-shaped portion 85 has a through-hole 87 penetrating through the plate-shaped portion 85 in the thickness direction D3. The plate-shaped portion 85 has a plurality (as one example, four) of the through-holes 87. The protruding portion 86 is a portion protruding in the third direction A3 from an end portion (lower right end in FIG. 4) of the plate-shaped portion 85 in the first direction A1.

When the main unit 46 is fixed to the sheet-shaped fabric 41, the electrical function portion 81 is disposed on the sheet-shaped fabric 41 (the inner fabric 44 or the outer fabric 45). FIG. 4 shows an example in which the electrical function portion 81 is disposed on a front surface (upper surface in FIG. 4) of the inner fabric 44. However, the electrical function portion 81 may be disposed on a back surface of the inner fabric 44 or the outer fabric 45. The disposition position of the electrical function portion 81 with respect to the sheet-shaped fabric 41 can be changed as appropriate. When the electrical function portion 81 is disposed on the front surface of the inner fabric 44, the attachment member 82 is disposed on the back surface (lower surface in FIG. 4) of the inner fabric 44. At this time, the plate-shaped portion 85 comes into contact with the back surface of the inner fabric 44. The plate-shaped portion 85 is disposed such that the protruding portion 86 protrudes in the first direction A1 from the end portion of the inner fabric 44 in the first direction A1 and protrudes in the third direction A3 (a direction in which the electrical function portion 81 is located when viewed from the plate-shaped portion 85, namely, upward in FIG. 4).

The screws 83 are inserted into the through-holes 87 along the third direction A3. For example, holes into which the screws 83 are inserted are formed in the sheet-shaped fabric 41 (the inner fabric 44 or the outer fabric 45). The screws 83 inserted into the through-holes 87 penetrate through the holes. In the example of FIG. 4, the electrical function portion 81 is disposed on the inner fabric 44 of the sheet-shaped fabric 41. For this reason, the through-holes 87 are formed in the inner fabric 44 of the sheet-shaped fabric 41, and the through-holes 87 are not formed in the outer fabric 45. When the electrical function portion 81 is disposed on the outer fabric 45, the through-holes 87 may be formed only in the outer fabric 45, or the through-holes 87 may be formed in both the inner fabric 44 and the outer fabric 45. The screws 83 inserted into the through-holes 87 are screwed into the screw grooves of the insertion holes of the housing 84. As a result, the inner fabric 44 is sandwiched between the electrical function portion 81 and the attachment member 82, so that the main unit 46 is fixed to the sheet-shaped fabric 41.

The configuration of the main unit 46 and the mode of fixing the main unit 46 to the sheet-shaped fabric 41 are not limited to those described above. The main unit 46 may not be fixed to the sheet-shaped fabric 41.

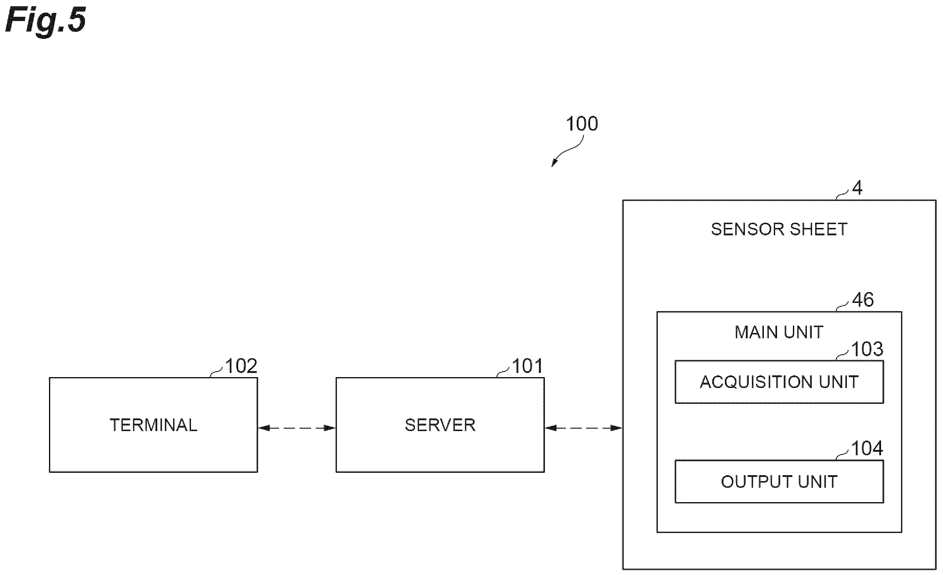

A sensing system 100 includes the sensor sheet 4 described above will be described. FIG. 5 is a block diagram showing the sensing system 100 according to one embodiment. The sensing system 100 includes, for example, the sensor sheet 4 described above, a server 101, and a terminal 102. The sensor sheet 4 and the server 101 are communicably connected to each other. The server 101 and the terminal 102 are communicably connected to each other. Communication between the sensor sheet 4 and the server 101 and communication between the server 101 and the terminal 102 may be realized, for example, by a wireless communication interface such as a wireless Local Area Network (LAN) or Bluetooth (registered trademark).

The main unit 46 of the sensor sheet 4 functionally includes an acquisition unit 103 and an output unit 104. The acquisition unit 103 acquires the vital data of the user acquired by the sensor unit 42 (see FIG. 2). The output unit 104 outputs the vital data acquired by the acquisition unit 103 to the server 101.

The server 101 outputs the vital data, which is output from the sensor sheet 4, to the terminal 102. For example, the terminal 102 analyzes the vital data acquired from the server 101. The terminal 102 displays the analyzed data toward the a user of the sensing system 100. The user of the sensing system 100 may be a person who senses the sleep of the user of the cushion body 1 using the sensing system 100, or may be a person who checks the analysis result of the vital data output from the sensor sheet 4. The user of the sensing system 100 may be the user of the cushion body 1 or may be a person different from the user of the cushion body 1.

The sensing system 100 may be, for example, software that can be downloaded to the terminal 102. In this case, the user of the sensing system 100 who downloads the software to the terminal 102 can display the analysis result of the vital data, which is output from the sensor sheet 4, on the terminal 102. The terminal 102 may be, for example, a smartphone, a tablet, or a personal computer. The vital data may be analyzed by the server 101. In this case, the terminal 102 displays data analyzed by the server 101. The user of the sensing system 100 acquires information regarding the heartbeat, breathing, and body movement of the user of the cushion body 1 during sleep by checking the data displayed on the terminal 102. The sensing system 100 may not include the server 101. The sensor sheet 4 and the terminal 102 may be directly communicably connected to each other.

Actions and effects of the sensor sheet 4 and the cushion body 1 will be described. As shown in FIGS. 1A, 1B, 2, and 3, the sensor sheet 4 and the cushion body 1 include the fixing portions 71 and 72 for fixing the sheet-shaped fabric 41 to the cover fabric 3. By fixing the sheet-shaped fabric 41 to the cover fabric 3 using the fixing portions 71 and 72, misalignment of the sensor sheet 4 when the body of the user of the cushion body 1 is placed on the cushion body 1 can be suppressed.

For example, when the thread-shaped sensor 47 is directly embroidered on the cover fabric 3, the thread-shaped sensor 47 cannot be easily removed from the cover fabric 3, and therefore, the cover fabric 3 cannot be washed. On the other hand, in the sensor sheet 4, the fixing portions 71 and 72 are attachable to and detachable from the cover fabric 3, and therefore, the sensor sheet 4 can be easily removed from the cover fabric 3 removed from the core material 2. Therefore, the cover fabric 3 can be easily washed.

The sensor sheet 4 includes the sheet-shaped fabric 41 and the thread-shaped sensor 47 embroidered on the sheet-shaped fabric 41. For example, one thread-shaped sensor 47 is attached by being embroidered such that the one thread-shaped sensor 47 spreads out two-dimensionally on the sheet-shaped fabric 41. A wide range of vital data can be acquired by the thread-shaped sensor 47, and the number of the thread-shaped sensors 47 can be reduced to one. As a result, an electrical system such as a connector can be simplified.

The core material 2 has the upper surface 21 on which the body of the user is placed; the lower surface 22 facing opposite the upper surface 21; and the side surfaces 23 connecting the upper surface 21 and the lower surface 22. The fixing portions 71 and 72 fix the sheet-shaped fabric 41 to the cover fabric 3 at positions between the side surfaces 23 of the core material 2 and the cover fabric 3. For example, when the fixing portions 71 and 72 fix the sheet-shaped fabric 41 to the cover fabric 3 at positions between the upper surface 21 on which the body of the user is placed and the cover fabric 3, there is a possibility that the fixing portions 71 and 72 come into contact with the body of the user when the body of the user is placed on the cushion body 1, thereby causing discomfort to the user. On the other hand, when the fixing portions 71 and 72 fix the sheet-shaped fabric 41 to the cover fabric 3 between the side surfaces 23 of the core material 2 and the cover fabric 3, causing discomfort to the user can be avoided.

For example, the core material 2 and the cover fabric 3 have a rectangular shape in a plan view. The cover fabric 3 includes the plurality of fixed portions 36 to which the fixing portions 71 and 72 are fixed. The plurality of fixed portions 36 are aligned along the longitudinal direction D1 of the cover fabric 3. When the body of the user is placed on the cushion body 1, the body of the user is placed along the longitudinal direction D1 of the cover fabric 3. When the plurality of fixed portions 36 are aligned along the longitudinal direction D1 of the cover fabric 3, the fixing position of the sensor sheet 4 can be arbitrarily changed along the longitudinal direction D1 of the cover fabric 3. Accordingly, for example, the position of the sensor sheet 4 in the longitudinal direction D1 of the cover fabric 3 can be adjusted according to the physique of the user. Furthermore, the position of the sensor sheet 4 can be freely changed depending on a location on the body from which vital data is to be measured. Since the sensor sheet 4 can be disposed at an appropriate position according to the user, the vital data of the user can be more accurately acquired.

For example, the fixing portions 71 and 72 include the snap buttons 73, and fix the sheet-shaped fabric 41 to the cover fabric 3 via the snap buttons 73. In this case, the sensor sheet 4 is more easily attachable to and detachable from the cover fabric 3 via the snap buttons 73. For example, compared to when the fixing portions 71 and 72 include hook-and-loop fasteners instead of the snap buttons 73, discomfort when the fixing portions 71 and 72 come into contact with the body of the user can be reduced.

For example, the sheet-shaped fabric 41 includes the inner fabric 44 on which the thread-shaped sensor 47 is embroidered, and the outer fabric 45 that accommodates the inner fabric 44. The outer fabric 45 is waterproof. The thread-shaped sensor 47 may be vulnerable to moisture, and in this case, when the thread-shaped sensor 47 comes into contact with water, there is a possibility that the detection accuracy of the thread-shaped sensor 47 decreases. When the inner fabric 44 on which the thread-shaped sensor 47 is embroidered is accommodated in the outer fabric 45 that is waterproof, the contact of the thread-shaped sensor 47 with moisture can be suppressed. Therefore, a decrease in the detection accuracy of the thread-shaped sensor 47 can be suppressed.

For example, the thread-shaped sensor 47 includes the curved portion 48 having a curved shape in a plan view. In the curved portion 48 at which the thread-shaped sensor 47 has a curved shape, the detection accuracy is higher than, for example, in a portion at which the thread-shaped sensor 47 has a linear shape. When the thread-shaped sensor 47 includes the curved portion 48 having a curved shape in a plan view, the detection accuracy of the thread-shaped sensor 47 can be improved.

The embodiment of the sensor sheet and the cushion body according to the present disclosure has been described above. However, the sensor sheet and the cushion body according to the present disclosure are not limited to the above-described embodiments, and can be changed as appropriate without departing from the concept described in the claims.

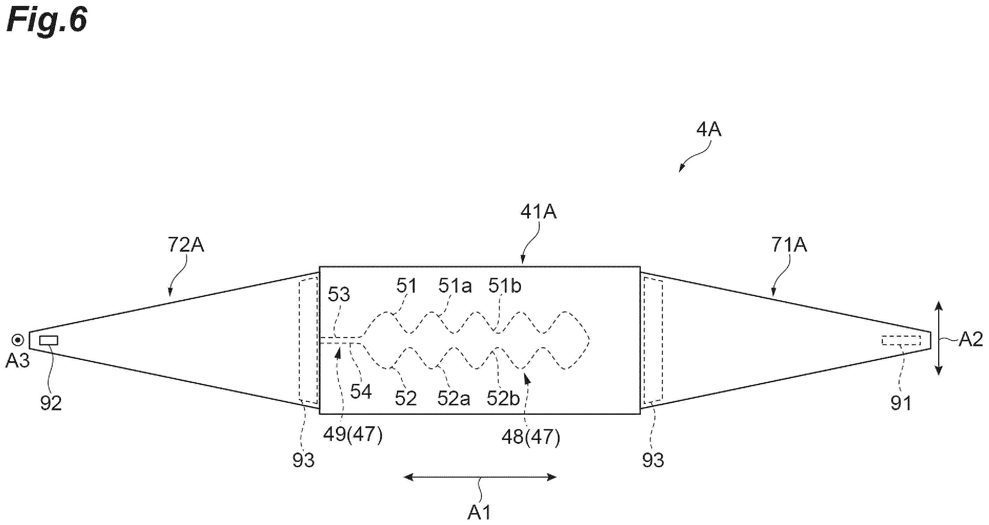

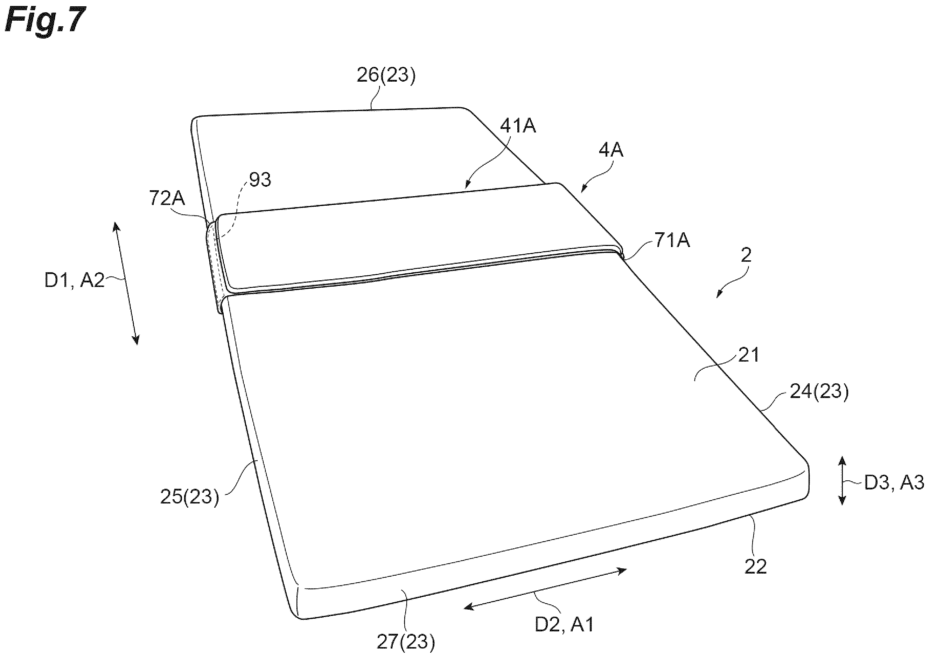

The sensor sheet 4 according to the above-described embodiment is attachable to and detachable from the inner side (back surface) of the cover fabric 3. However, the sensor sheet may be attachable to and detachable from the outer side (front surface) of the cover fabric, or may be attachable to and detachable from the core material. Hereinafter, a sensor sheet 4A according to a modification example will be described. FIG. 6 is a schematic plan view showing the sensor sheet 4A according to the modification example. In FIG. 6, the illustration of the sensor unit 42 and the power supply unit 43 among the configurations of the sensor sheet 4A is omitted. FIG. 7 is a schematic perspective view showing the core material 2 to which the sensor sheet 4A shown in FIG. 6 is attached. In the following description, the differences from the sensor sheet 4 described above will be mainly described, and duplicate descriptions will be omitted as appropriate with the same or corresponding elements denoted by the same reference signs.

The sensor sheet 4A includes a sheet-shaped fabric 41A having a form different from that of the sheet-shaped fabric 41. A length of the sheet-shaped fabric 41A in the first direction A1 is substantially the same as, for example, the length of the core material 2 in the lateral direction D2. The length of the sheet-shaped fabric 41A in the first direction A1 is, for example, 20 cm or more and 200 cm or less. A length of the sheet-shaped fabric 41A in the second direction A2 is, for example, 10 cm or more and 70 cm or less.

Similarly to the above description, the sensor sheet 4A includes the thread-shaped sensor 47 embroidered on the sheet-shaped fabric 41A. The thread-shaped sensor 47 is a single sensor, and one thread-shaped sensor 47 is fixed to spread out two-dimensionally on the sheet-shaped fabric 41A. The thread-shaped sensor 47 includes the curved portion 48 and the linear portion 49. The curved portion 48 has a wave shape in a plan view.

The sensor sheet 4A includes fixing portions 71A and 72Ahaving a form different from that of the fixing portions 71 and 72. The fixing portion 71A and the fixing portion 72A (another fixing portion) fix the sheet-shaped fabric 41A to the core material 2. The fixing portions 71A and 72A are attachable to and detachable from the core material 2. The fixing portions 71A and 72A are belt-shaped member extending in the first direction A1. The fixing portions 71A and 72A have, for example, an isosceles trapezoidal shape. The length of each of the fixing portions 71A and 72A in the second direction A2 decreases as each of the fixing portions 71A and 72A extends away from the sheet-shaped fabric 41A.

Each of the fixing portions 71A and 72A is made of, for example, a stretchable mesh material. In this case, since the fixing portions 71A and 72A are stretchable, the sensor sheet 4A can be easily moved, and the breathability of the fixing portions 71A and 72A can be enhanced. However, the constituent material of the fixing portions 71A and 72A may not be stretchable, and is not particularly limited. The shape of the fixing portions 71A and 72A may be, for example, a rectangular shape, and is not limited to an isosceles trapezoidal shape.

For example, a length of each of the fixing portions 71A and 72A in the first direction A1 is shorter than the length of the sheet-shaped fabric 41A in the first direction A1. For example, the length of the fixing portion 71A in the first direction A1 and the length of the fixing portion 72A in the first direction A1 are the same. For example, the sensor sheet 4A is attached to the core material 2 so as to be wrapped around the core material 2. The lengths of the fixing portions 71A and 72A in the first direction A1 may be different from each other, and should be of such a length that the fixing portions 71A and 72A can be wrapped around the core material 2.

The fixing portion 71A is connected to one end (right end in FIG. 6) of the sheet-shaped fabric 41A in the first direction A1. The fixing portion 71A includes a first fixing member 91. The first fixing member 91 is provided on one end side (right end side in FIG. 6) of the fixing portion 71A in the first direction A1. The first fixing member 91 is, for example, a loop (female part) of a hook-and-loop fastener.

The fixing portion 72A is connected to the other end (left end in FIG. 6) of the sheet-shaped fabric 41A in the first direction A1. The fixing portion 72A includes a second fixing member 92. The second fixing member 92 is provided on the other end side (left end side in FIG. 6) of the fixing portion 72A in the first direction A1. The second fixing member 92 is, for example, a hook (male part) of a hook-and-loop fastener.

The first fixing member 91 and the second fixing member 92 are not limited to a hook-and-loop fastener. The first fixing member 91 and the second fixing member 92 may be, for example, slide fasteners or snap buttons. When the first fixing member 91 and the second fixing member 92 are snap buttons, for example, the first fixing member 91 is a female part of the snap button, and the second fixing member 92 is a male part of the snap button.

The first fixing member 91 and the second fixing member 92 enable the fixing portion 71A to be attached to and detached from the fixing portion 72A. The fixing portions 71A and 72A are fixed to each other via the first fixing member 91 and the second fixing member 92. For example, a length of the first fixing member 91 in the first direction A1 is longer than a length of the second fixing member 92 in the first direction A1.

Each of the fixing portions 71A and 72A includes a support 93 at a portion adjacent to the sheet-shaped fabric 41A. The support 93 of the fixing portion 71A is provided at an end portion of the fixing portion 71A on the side opposite the first fixing member 91. The support 93 of the fixing portion 72A is provided at an end portion of the fixing portion 72A on the side opposite the second fixing member 92.

The support 93 is made of a material that is less likely to deform than the constituent material of the fixing portions 71A and 72A. For example, a hardness of the support 93 is higher a hardness of the fixing portions 71A and 72A. For example, a measurement method specified in JIS K7215:1986 may be used to measure the hardness of the support 93, the fixing portion 71A, and the fixing portion 72A. The support 93 has, for example, an isosceles trapezoidal shape. The supports 93 are formed, for example, along the shape of the end portions of the fixing portions 71A and 72A that are closer to the sheet-shaped fabric 41A. In this case, the support 93 has a trapezoidal shape. The supports 93 are disposed such that lower bases of the supports 93 extend along lower bases of the fixing portions 71A and 72A having a trapezoidal shape and the legs of the trapezoids of the supports 93 extend along the legs of the trapezoids of the fixing portions 71A and 72A.

As shown in FIG. 7, the sensor sheet 4A is used while being attached to the core material 2. A method for attaching the sensor sheet 4A to the core material 2 will be described. First, the sheet-shaped fabric 41A of the sensor sheet 4A is disposed on the upper surface 21 of the core material 2. For example, the sheet-shaped fabric 41A is disposed on the upper surface 21 in a state where the first direction A1 of the sheet-shaped fabric 41A is set to coincide with the lateral direction D2 of the core material 2.

As described above, the length of the sheet-shaped fabric 41A in the first direction A1 may be substantially the same as the length of the core material 2 in the lateral direction D2. In this case, the sheet-shaped fabric 41A is disposed across the entirety of the core material 2 in the lateral direction D2 in a state where the sheet-shaped fabric 41A is disposed on the upper surface 21 of the core material 2. In a state where the sheet-shaped fabric 41A is disposed on the upper surface 21 of the core material 2, the fixing portions 71A and 72A are disposed on the lower surface 22 of the core material 2. At this time, each of the fixing portions 71A and 72A is folded downward with respect to the sheet-shaped fabric 41A, and each of the folded fixing portions 71A and 72A is moved below the lower surface 22. The fixing portions 71A and 72A are fixed to each other on the lower surface 22 of the core material 2. The hook (second fixing member 92) of the fixing portion 72A locks to the loop (first fixing member 91) of the fixing portion 71A. Accordingly, the fixing portions 71A and 72A are fixed to each other via the first fixing member 91 and the second fixing member 92.

In a state where the sensor sheet 4A is attached to the core material 2, the support 93 of the fixing portion 71A faces the first side surface 24 of the core material 2. The support 93 of the fixing portion 72A faces the second side surface 25 of the core material 2. As described above, the lengths of the fixing portions 71A and 72A in the first direction A1 may be the same. The fixing portions 71A and 72A are fixed to each other at substantially the center of the lower surface 22 of the core material 2 in the lateral direction D2. The sensor sheet 4A is attached to the core material 2 so as to be wrapped around the core material 2. The core material 2 to which the sensor sheet 4A is attached through the above-described steps is accommodated in the cover fabric, thereby completing the cushion body to which the sensor sheet 4A is attached.

Actions and effects of the sensor sheet 4A will be described. The sensor sheet 4A includes the fixing portions 71A and 72A for fixing the sheet-shaped fabric 41A to the core material 2. By fixing the sheet-shaped fabric 41A to the core material 2 using the fixing portions 71A and 72A, misalignment of the sensor sheet 4A when the body of the user of the cushion body is placed on the cushion body can be suppressed. Since the fixing portions 71A and 72A in the sensor sheet 4A are attachable to and detachable from the core material 2, the sensor sheet 4A can be easily removed from the core material 2 removed from the cover fabric.

The sensor sheet 4A includes the sheet-shaped fabric 41A and the thread-shaped sensor 47 embroidered on the sheet-shaped fabric 41A. A wide range of vital data can be acquired by the thread-shaped sensor 47, and the number of the thread-shaped sensors 47 can be reduced. As a result, an electrical system such as a connector can be simplified.

When the cushion body including the sensor sheet 4A is placed on a placement surface (for example, a floor surface or the like), a back surface of the cushion body (a surface located on the lower surface 22 side of the core material 2) comes into contact with the placement surface. At this time, the fixing portions 71A and 72A are sandwiched between the lower surface 22 of the core material 2 and the placement surface, so that unintended movement of the sensor sheet 4A can be suppressed. Therefore, misalignment of the sensor sheet 4A can be more reliably suppressed.

In the sensor sheet 4A, the fixing portions 71A and 72A extending from both ends of the sheet-shaped fabric 41A in the first direction A1 are detachably fixed to each other via the first fixing member 91 and the second fixing member 92. The fixing portions 71A and 72A are more firmly fixed to the core material 2 by the first fixing member 91 and the second fixing member 92.

The sensor sheet 4A can be easily moved to a desired location on the core material 2 by removing the first fixing member 91 and the second fixing member 92. Since the sensor sheet 4A can be easily moved to a desired location on the core material 2, for example, the position of the sensor sheet 4A in the longitudinal direction D1 of the core material 2 can be adjusted according to the physique of the user.

For example, when the cushion body is used for a long period of period with the head of the user placed on one end side of the cushion body in the longitudinal direction D1, there is a possibility that the amount of sinking of the one end side becomes excessive over time. In this case, the cushion body may be used with the head of the user placed on the other end side of the cushion body in the longitudinal direction D1. Even when the cushion body is used in this manner, the sensor sheet 4A can be easily moved to a desired location on the core material 2. Therefore, for example, the position of the sensor sheet 4A in the longitudinal direction D1 of the core material 2 can be easily adjusted according to a direction in which the body of the user placed on the cushion body extends.

In the sensor sheet 4A, the length of the first fixing member 91 in the first direction A1 is longer than the length of the second fixing member 92 in the first direction A1. In this case, the fixing position of the second fixing member 92 with respect to the first fixing member 91 can be arbitrarily changed along the first direction A1. Since the length of the fixing portions 71A and 72A wrapped around the core material 2, namely, the length of portions of the fixing portions 71A and 72A that are wrapped around the core material 2 can be freely changed, the sensor sheet 4A can be attached to cushion bodies of various sizes.

When the body of the user is placed on the sensor sheet 4A, there is a possibility that the sensor sheet 4A is crushed in the thickness direction D3 due to the weight of the user. At this time, the sensor sheet 4A may stretch in the lateral direction D2 of the core material 2, and therefore, gaps may occur between the side surfaces 23 of the core material 2 and the fixing portions 71A and 72A. As a result, the fixing of the sensor sheet 4A by the fixing portions 71A and 72A becomes insufficient, and therefore, there is a possibility that the sensor sheet 4A is misaligned.

On the other hand, in the sensor sheet 4A, each of the fixing portions 71A and 72A includes the support 93. In a state where the sensor sheet 4A is attached to the core material 2, the support 93 faces the side surface 23 of the core material 2. Accordingly, when the body of the user is placed on the sensor sheet 4A, the deformation of the fixing portions 71A and 72A on the side surfaces 23 of the core material 2 can be suppressed. As a result, the occurrence of gaps between the side surfaces 23 of the core material 2 and the fixing portions 71A and 72A can be suppressed, and misalignment of the sensor sheet 4A can be more reliably suppressed.

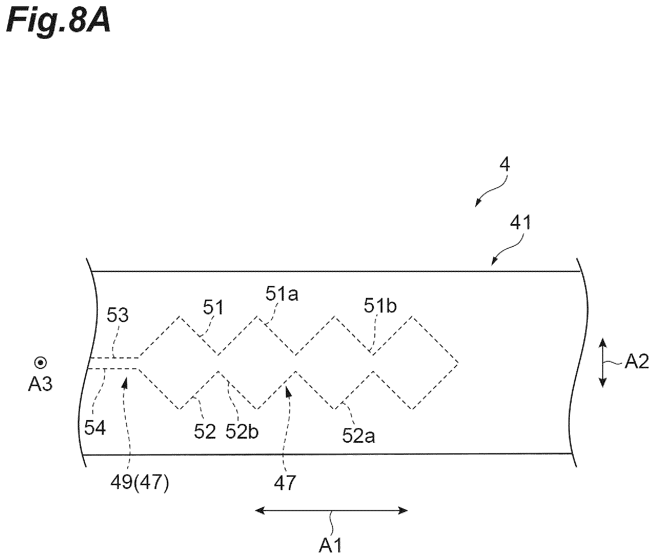

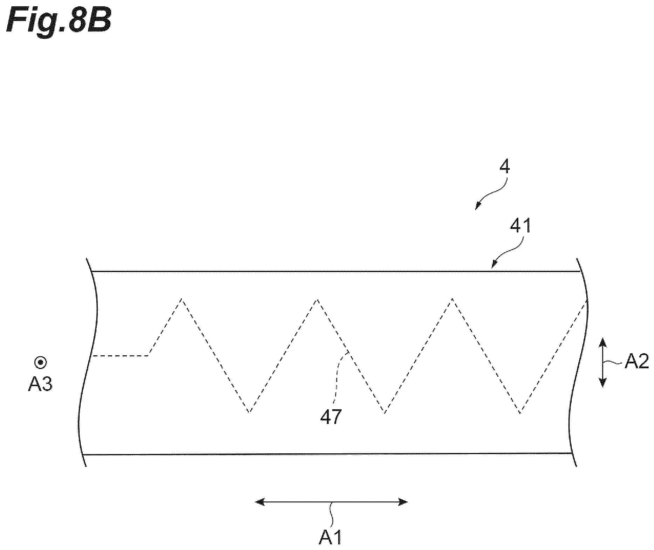

The modification example of the sensor sheet and the cushion body according to the present disclosure has been described above. However, the sensor sheet and the cushion body according to the present disclosure can be further modified. For example, an example in which the curved portion 48 of the thread-shaped sensor 47 has a wave shape has been described above. However, the shape of the thread-shaped sensor 47 is not limited to a wave shape. For example, the thread-shaped sensor 47 may not include curved portion 48. As shown in FIG. 8A, the thread-shaped sensor 47 may have a triangular wave shape. In this case, the first wave-shaped portion 51 and the second wave-shaped portion 52 aligned in the second direction A2 are formed by the thread-shaped sensor 47 that is linearly disposed. The thread-shaped sensor 47 may have a rectangular wave shape or a diamond wave shape. As shown in FIG. 8B, the thread-shaped sensor 47 may have a sawtooth shape. In this case, the thread-shaped sensor 47 does not include two wave-shaped portions aligned in the second direction A2, but include one wave-shaped portion. The wave-shaped portion is formed by the thread-shaped sensor 47 that is linearly disposed.

An example in which the sensor sheet 4 includes the fixing portions 71 and 72 for fixing the sheet-shaped fabric 41 to the cover fabric 3 has been described above. However, the fixing portions 71 and 72 may fix the sheet-shaped fabric 41 to the core material accommodated in the cover fabric. The fixing portions 71 and 72 may be attachable to and detachable from the core material. In this case, the fixing portions 71 and 72 may fix the sheet-shaped fabric 41 to the core material via the snap buttons 73. When the fixing portions 71 and 72 fix the sheet-shaped fabric 41 to the core material, the core material 2 of the above-described embodiment may include the fixed portions 36 on the first side surface 24 and the second side surface 25 of the core material 2. For example, the plurality of fixed portions 36 may be aligned in the longitudinal direction D1 of the core material 2.

In the above-described embodiment, an example in which when the sensor sheet 4 is attached to the cushion body 1, the sensor sheet 4 is disposed between the upper surface 21 of the core material 2 and the front fabric 31 of the cover fabric 3 has been described. When the sensor sheet 4 is disposed between the upper surface 21 of the core material 2 and the front fabric 31 of the cover fabric 3, the detection accuracy of the sensor sheet 4 can be improved. However, the sensor sheet 4 may be disposed between the lower surface 22 of the core material 2 and the back fabric 32 of the cover fabric 3.

An example in which the fixing portions 71 and 72 include the snap buttons 73 has been described above. However, the fixing portions 71 and 72 may include fixing members other than the snap buttons 73. For example, the fixing portions 71 and 72 may include at least one of hook-and-loop fasteners and slide fasteners instead of the snap buttons 73. In this case, the core material 2 or the cover fabric 3 may include hook-and-loop fasteners and slide fasteners as the fixed portions 36.

An example in which each of the fixing portions 71A and 72A of the sensor sheet 4A includes the support 93 has been described above. However, each of the fixing portions 71A and 72A may not include the support 93. An example in which the fixing portion 71A includes the first fixing member 91, and the fixing portion 72A includes the second fixing member 92 has been described above. However, the fixing portions 71A and 72A may not include a fixing member (the first fixing member 91 or the second fixing member 92). In this case, the fixing portions 71A and 72A may fix the sheet-shaped fabric 41A to the core material 2 by being sandwiched between the lower surface 22 of the core material 2 and the placement surface.

An example in which the length of the fixing portions 71A and 72A is of such a length that the fixing portions 71A and 72A can be wrapped around the core material 2 has been described above. However, when the fixing portions 71A and 72A fix the sensor sheet 4A to the core material 2 by being sandwiched between the lower surface 22 of the core material 2 and the placement surface, the length of the fixing portions 71A and 72A may be shorter than that in the above-described modification example. More specifically, the length of the fixing portions 71A and 72A may be of such a length that the sheet-shaped fabric 41A can be disposed on the upper surface 21 of the core material 2 and then the fixing portions 71A and 72A can be sandwiched between the lower surface 22 of the core material 2 and the placement surface.

An example in which the sheet-shaped fabric 41 has a rectangular shape in a plan view has been described above. Furthermore, an example in which the sheet-shaped fabric 41A has a rectangular shape in a plan view has been described above. However, the shape of the sheet-shaped fabrics 41 and 41A should be a sheet shape, and is not limited to a rectangular shape. For example, the sheet-shaped fabrics 41 and 41A may have, in a plan view, a major axis extending in the longitudinal direction, and a minor axis extending in the lateral direction and intersecting the major axis. The sheet-shaped fabrics 41 and 41A may have, for example, an oval shape extending in the longitudinal direction, or a polygonal shape other than a rectangular shape.

### Reference Signs List

1: cushion body, 2: core material, 3: cover fabric, 4, 4A: sensor sheet, 21: upper surface, 22: lower surface, 23: side surface, 24: first side surface, 25: second side surface, 26: third side surface, 27: fourth side surface, 31: front fabric, 32: back fabric, 33: opening and closing member, 34: slide member, 35: opening and closing portion, 36: fixed portion, 41, 41A: sheet-shaped fabric, 42: sensor unit, 43: power supply unit, 44: inner fabric, 45: outer fabric, 46: main unit, 47: thread-shaped sensor, 48: curved portion, 49: linear portion, 51: first wave-shaped portion, 51a, 52a: peak portion, 51b, 52b: valley portion, 52: second wave-shaped portion, 53: first linear portion, 54: second linear portion, 61: power cord, 62: power plug, 63: connector, 71, 71A, 72: fixing portion, 72A: fixing portion (another fixing portion), 73: snap button, 81: electrical function portion, 82: attachment member, 83: screw, 84: housing, 84a: socket, 85: plate-shaped portion, 86: protruding portion, 87: through-hole, 91: first fixing member, 92: second fixing member, 93: support, 100: sensing system, 101: server, 102: terminal, 103: acquisition unit, 104: output unit, A1: first direction (longitudinal direction), A2: second direction (lateral direction), A3: third direction, D1: longitudinal direction, D2: lateral direction, D3: thickness direction, L: center line.

## Claims

1. A sensor sheet that is attached to a cushion body including a core material made of a flexible material and a cover fabric that accommodates the core material and that is attachable to and detachable from the core material, and that acquires vital data of a user of the cushion body, comprising:
a sheet-shaped fabric;
a thread-shaped sensor embroidered on the sheet-shaped fabric; and
a fixing portion that fixes the sheet-shaped fabric to at least one of the core material and the cover fabric,
wherein the fixing portion is attachable to and detachable from the at least one of the core material and the cover fabric.

2. The sensor sheet according to claim 1,
wherein the core material has an upper surface on which a body of the user is placed, a lower surface facing opposite the upper surface, and side surfaces connecting the upper surface and the lower surface, and
the fixing portion fixes the sheet-shaped fabric to the cover fabric at positions between the side surfaces of the core material and the cover fabric.

3. The sensor sheet according to claim 2,
wherein the core material and the cover fabric have a rectangular shape in a plan view,
the cover fabric includes a plurality of fixed portions to which the fixing portion is fixed, and
the plurality of fixed portions are aligned along a longitudinal direction of the cover fabric.

4. The sensor sheet according to claim 1 or 2,
wherein the fixing portion includes snap buttons, and fixes the sheet-shaped fabric to the at least one of the core material and the cover fabric via the snap buttons.

5. The sensor sheet according to any one of claims 1 to 3,
wherein the sheet-shaped fabric includes an inner fabric on which the thread-shaped sensor is embroidered, and an outer fabric that accommodates the inner fabric, and
the outer fabric is waterproof.

6. The sensor sheet according to any one of claims 1 to 3,
wherein the thread-shaped sensor includes a curved portion having a curved shape in a plan view.

7. The sensor sheet according to claim 1 or 2, further comprising:
another fixing portion different from the fixing portion,
wherein the sheet-shaped fabric has, in a plan view, a major axis extending in a longitudinal direction, and a minor axis extending in a lateral direction and intersecting the major axis,
the fixing portion is connected to one end of the sheet-shaped fabric in the longitudinal direction,
the other fixing portion is connected to the other end of the sheet-shaped fabric in the longitudinal direction,
the core material has an upper surface on which a body of the user is placed, a lower surface facing opposite the upper surface, and side surfaces connecting the upper surface and the lower surface, and
in a state where the sheet-shaped fabric is disposed on the upper surface of the core material, the sheet-shaped fabric is fixed to the core material by fixing the fixing portion and the other fixing portion to each other on the lower surface of the core material.

8. The sensor sheet according to claim 7,
wherein the fixing portion includes a first fixing member provided on one end side of the fixing portion in the longitudinal direction,
the other fixing portion includes a second fixing member provided on the other end side of the other fixing portion in the longitudinal direction, and
the fixing portion and the other fixing portion are fixed to each other via the first fixing member and the second fixing member.

9. The sensor sheet according to claim 8,
wherein each of the fixing portion and the other fixing portion includes a support at a portion adjacent to the sheet-shaped fabric,
the support of the fixing portion is provided at an end portion of the fixing portion on a side opposite the first fixing member,
the support of the other fixing portion is provided at an end portion of the other fixing portion on a side opposite the second fixing member, and
in a state where the sensor sheet is attached to the core material, the support of the fixing portion and the support of the other fixing portion face the side surfaces of the core material.

10. A cushion body, comprising:
the sensor sheet according to any one of claims 1 to 3;
the core material; and
the cover fabric that accommodates the core material.
